# EUROPEAN PATENT APPLICATION

(11) **EP 2 669 830 A1**
(43) Date of publication of application: **04.12.2013**
(21) Application number: 12170525.5
(22) Date of filing: 01.06.2012
(51) Int. Cl.: G06F 19/00

(54) **Preparation and display of derived series of medical images**

(71) Applicant: Kabushiki Kaisha Toshiba, Inc., Tokyo 105-8001 (JP); Toshiba Medical Systems Corporation, Otawara-shi, Tochigi-ken 324-8550 (JP)
(72) Inventor: Papageorgiou, Pavlos, Edinburgh, EH6 5NP (GB); Smout, Andrew Michael Christian, Edinburgh, EH6 5NP (GB); Cruickshank, Kristopher Thomas, Edinburgh, EH6 5NP (GB)
(74) Representative: Lewis, Darren John

(57) **Abstract**

Certain embodiments provide a computer system for displaying original and derived images and image series, each derived image series comprising a sequence of image frames. The computer system comprises an application component operable to create a specification for generation of a derived image series based on user interface interaction with displayed original or derived images, each derived image series specification providing values for a plurality of visualization parameters including a parameter whose value varies from frame to frame within the derived image series and a plurality of parameters whose values are fixed from frame to frame of the derived image series; and generate a derived image series by manipulating the medical image data set according to the derived image series specification. The display is configured to receive from the application component and display the derived image series generated from the derived image series specification.

## Description

### BACKGROUND OF THE INVENTION

Embodiments described herein generally relate to preparation and display of a derived series of medical images from image data sets in a client-server environment.

A state of the art hospital network is generally referred to as a Picture Archiving and Communication System (PACS). A PACS is used as a store for volume data representing diagnostic images of different types in a digital format organized in a single central archive. The most common image data format currently employed for medical applications is the "Digital Imaging and Communications in Medicine" (DICOM) format. In the medical field, two-dimensional (2D) and three-dimensional (3D) image data sets are collected by a variety of techniques and types of acquisition system - referred to as modalities in the field.

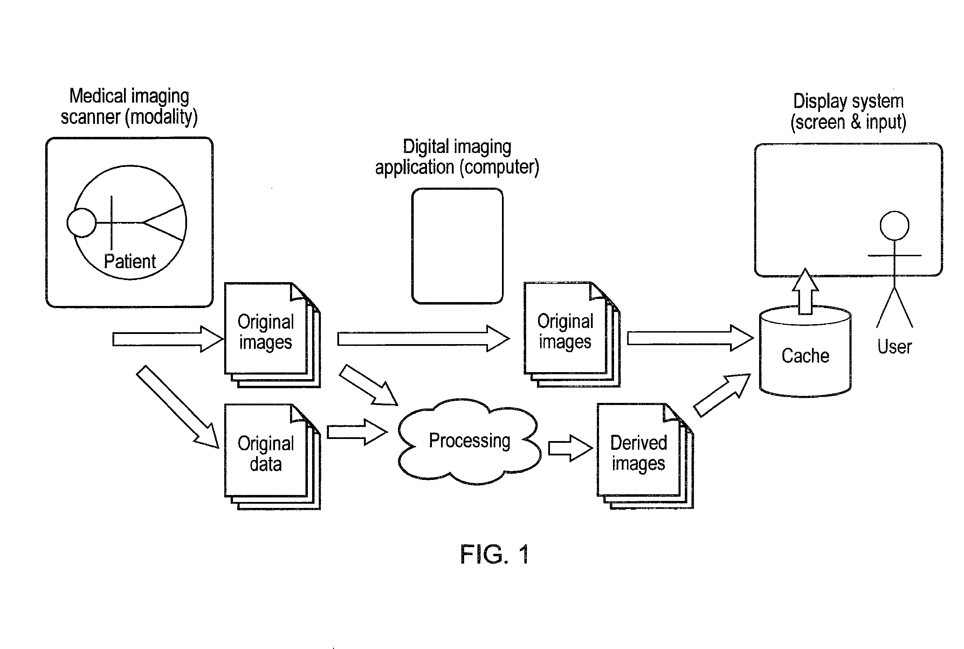

Figure 1 is a schematic drawing of principal elements of a medical imaging product chain. Initially a patient is scanned using an imaging device to perform computer-assisted tomography (CT), magnetic resonance (MR), digital radiography (DR), computed radiography (CR), positron-emission-tomography (PET), ultrasound or other modality. A modality generates two types of data. The first type of data, labeled original images, are images generated by the modality and are intended to be viewed directly by humans, typically radiologists or other medical professionals. Examples of original images are X-rays, CT cross-sectional images, MR sectional images, ultrasound fan images etc. The second type of data, labeled original data, is data that is not produced in the form of an image, or is composed of a very large collection of images, and is not intended to be viewed by humans directly, or in the aggregate. Original data may simply be an aggregation of images in a file, or it may be a three-dimensional or multi-dimensional representation of data that is beyond the domain of images.

The original images and/or original data generated by a modality are transferred to a computer workstation where the original images are locally cached, i.e. held in a memory cache at the workstation. The workstation hosts a digital imaging software application which is used to display the original images to a medical imaging professional on a display system, such as a high resolution monitor or collection of monitors. The digital imaging application may also pass some of the original images directly onwards, for example to other applications on the same computer or computer network, or over a network to other computer systems.

The digital imaging application may process some combination of original images and/or original data to generate derived images. Derived images are images that are different from original images and are intended to be seen directly by a medical imaging professional such as a radiologist. Examples of derived images are:
1. CT or MR sectional images that are are sectioned on different planes than the original images, or on planes that the CT scanner would not be capable of sectioning;
2. CT or MR sectional images that represent thicker sections (zones) of anatomy than the original images and are produced from resampling and summation of original images or data;
3. monochrome or color projection images that are derived from CT, MR, or Ultrasound data by assigning optical properties to the content of original images or original data and then performing an approximate simulation of lighting and projection through the data (a technique known as volume rendering);
4. cross sectional images that are sampled on curved surfaces from data in the space defined by original images or original data; and
5. images derived using a combination of the above techniques, and or/other techniques.

It will be appreciated that the original images and/or original data may be a large amount of data, and that the imaging application may need a large amount of processing power to generate derived images in a timely manner.

The derived images are transferred to the computer workstation where the derived images are locally cached. In the prior art, caching has been used for improved handling of derived images in the following ways.

Reactive caching: This is the simplest form of caching. When a user requests a derived image series, the images making up the series are sent to the client and stored in a local cache. Once in the cache, the images are available immediately.

Static caching: An initial series of derived images is cached, this set being what the software, or a human technician, predicts the user will need. This works well when the user only references that set, but is ineffective when the user asks for a new series of derived images.

**Predictive caching:** The user is allowed to control the generation of derived images freely, and it is attempted to predict what the user will do next, by examining current and recent image generation parameters and the likely values of future image generation parameters. For example, if the user appears to be moving a sectioning plane in a left-right direction at 3.5mm per second (or per step), it is predicted that the plane will be 3.5mm further to the right in one second (or in the next step), so the relevant derived images are transmitted and cached locally in advance. In practice, these systems make successful predictions too rarely to be effective, and only predict parameter changes in the immediate neighborhood of current parameters.

**Historical caching:** Historical caching is effectively a form of reactive caching. The system simply caches all the derived images that the user has recently seen, so that they are available immediately if needed again. These systems obviously require the user to scan through all the images once in a manual operation before they are available in the cache. However, a good implementation of historical caching has proven to be more satisfactory to the user than static or predictive caching.

JP 2001-101060 (Toshiba Corp - Okuda Kenichi et al) and US 2004/0249806 A1 (=JP2000-0096559) (Fuji Photo Film Co Ltd - Shoji Kanada) disclose example prior art file retrieval and caching approaches from a Picture Archiving and Communication System (PACS) archive.

The derived images can then be viewed on the display system by a radiologist, cardiologist, surgeon, or other specialist. The purpose of displaying the original and the derived images is to make clinical decisions regarding the patient such as diagnosis, assessment of disease, treatment planning, assessment of treatment, and other such tasks.

Derived images are classified into different image types. For example, typical image types of a medical imaging product are planar MPR images, curved MPR images, 3D volume rendered images, simulated endoscopic images, etc.

Often combinations of original and derived images are displayed together on the display system, e.g. on different individual displays, if the workstation has 2, 3 or 4 displays and/or on different portions of the same display, e.g. 2 x 2, 2 x 3 or 3 x 2 arrays of image tiles. The particular arrangement of multiple complementary images obtained from the same or related sets of image data is referred to as a hanging protocol in the art.

When digital imaging in medicine was first deployed, the product chain offered only original images. Modalities were designed to produce human-readable images in manageable (low) quantity, and medical professionals were trained to read those images directly. In recent years modalities have improved technically such that, although they are still nominally seen as devices that produce human-readable images, they can produce such high volumes of images that they outstrip the capacity of human professionals to interpret them directly. The product chain is therefore expected to provide derived images as a means to summarize, reduce, or otherwise digest the original images created by modalities.

A common example of a set of derived images is the set of images generated from a volume data set using slab multi-planar reformatting (MPR). In this technique, MPR data are generated by taking a coordinate in view space, transforming the coordinate into volume space, and resampling the volume data using some form of interpolation to generate a new MPR data value for the discrete view space coordinate. An MPR slice is formed by carrying this out for a plurality of {x, y} coordinates at a fixed z value. If this is repeated for multiple values of z, then multiple MPR slices are determined and these slices can be projected to form an MPR slab. The MPR slab thus comprises a set of MPR slices which are aligned parallel to the image plane and disposed at different positions along the Z-axis.

In slab MPR, the slab can be gradually "moved" along the view axis in small increments by the user, so that the user sees a succession of images or frames projected from the volume data, where each frame relates to a projection through a slab occupying a location incrementally different from the previous slab. In use, a radiologist will "scroll" the slab back and forth interactively. For example, in a CT scan of a human head, a radiologist might take several seconds to traverse the head from top to bottom or back to front, with the traverse involving several hundred frames. A typical technique for generating such image frames is casting a ray through the volume data set for each pixel in an image and sampling the volume data sets at a number of discrete points along each respective ray. If using maximum intensity projection (MIP), the maximum voxel value or interpolated value along the ray within the slab is then selected. The selected maximum sample or interpolated value within the slab is taken to be the pixel value associated with its respective ray.

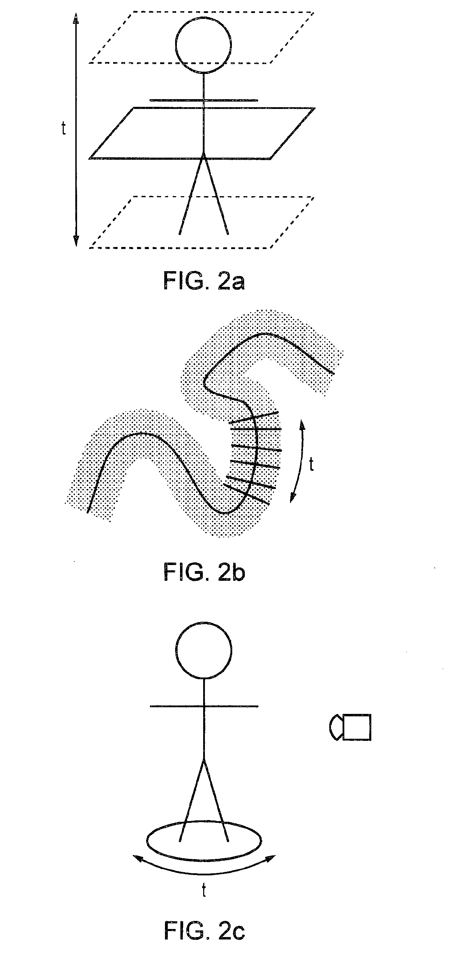

Figures 2a and 2b show examples of derived MPR image sequences. Figure 2a shows parallel multiplanar reformatting (MPR). Figure 2b shows MPR that is anchored to and locally perpendicular to a curve (cross-curve MPR).

Figure 2a shows a parallel MPR example where two positional coordinates and three orientation angles of the sectioning plane are fixed, and the plane is moved along its normal vector by varying parameter t, for example with the scroll wheel of a mouse. Typically there are minimum and maximum values for parameter t based on the data set. Different choices of position and orientation give rise to different parallel MPR sequences.

Figure 2b shows a cross-curve MPR example where a curve is defined through the patient's body, usually corresponding with an anatomical structure such as a vessel. The plane is moved by varying parameter t so that a given point on the plane moves along the curve, and the other degrees of freedom are constrained so that the plane is perpendicular to the curve at the bound point. The boundary values for parameter t are limited to a particular length portion of the vessel, for example. Different curves result in different cross-curve MPR sequences.

Figure 2c shows a further example of a rotational volume rendered projection (3D movie) or a rotational intensity projection (MIP movie). These are modeled by choosing an axis, often the patient's head-foot axis, and virtually rotating the patient while a virtual camera that is positioned to look at the rotation axis generates the projected images. Since the variable parameter t is angle, it does not have minimum and maximum limits, but rather loops round cyclically. Different choices of patient axis and inclination of the camera relative to that axis generate different rotational sequences.

Other examples of derived image sequences useful in medical imaging include, but are not limited to:
- Radial MPR sequences formed by rotating the sectioning plane around an axis;
- Curved surface MPR sequences formed by rotating the extrusion vector that defines the surface;
- Virtual endoscopic images formed by a virtual camera that moves along a curve looking along the curve;
- Local MPR sequences where a point on the sectioning plane is bound to a curve and the plane is constrained to be tangential to the curve at the bound point; and
- Local 3D or MIP sequences rendered by a camera that moves parallel to a curve, looking at a point on the curve

The above examples serve to illustrate the concept of image sequences.

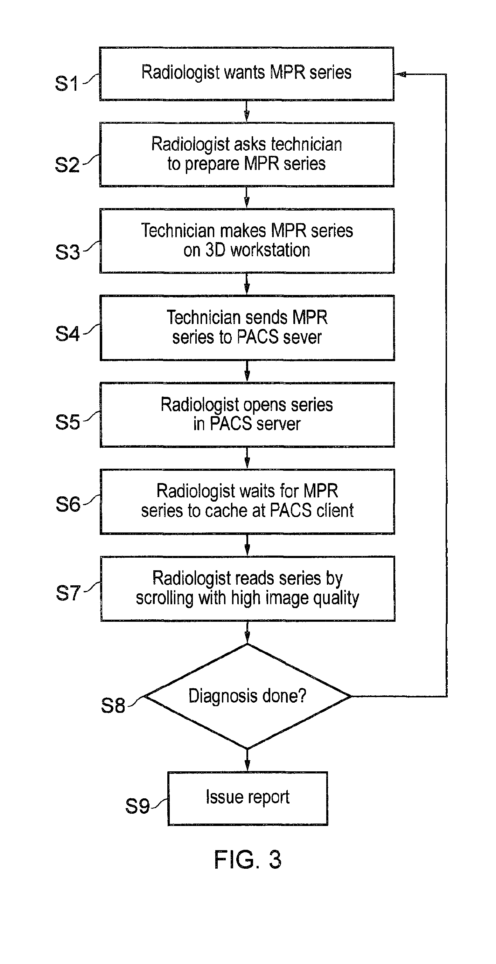

Figure 3 shows a standard workflow for preparing a sequence of derived images for MPR, such as would be applicable to the MPR examples of Figures 2a and 2b. This example workflow is a specific example of the general derived image workflow shown in Figure 1.

In Step S1, the sequence initiates with a radiologist wanting to have an MPR series prepared for his or her analysis.

In Step S2, the radiologist requests a technician to prepare the desired MPR series for a patient from a volume data set of the patient's scan.

In Step S3, the technician loads the patient data into a 3D visualization application to process the volume data set on a 3D workstation, i.e. a computer running a 3D visualization application, to prepare an MPR series conforming to the radiologist's specification. The MPR series is a set of derived images.

In Step S4, the technician sends the MPR series to the radiologist by sending a message over the network pointing the radiologist to the MPR series file stored on the PACS archive.

In Step S5, the radiologist opens the series stored on the PACS from his or her local machine, i.e. the LAN or web client computer.

In Step S6, the radiologist waits for the MPR series to cache at the client, i.e. waits for the MPR series data to transfer over the LAN or internet.

In Step S7, the radiologist reads or views the series by scrolling interactively up and down by varying the parameter t in order to conduct a diagnostic analysis. Of course, the image quality must be sufficient to permit reliable diagnosis on the client machine.

In Step S8, the radiologist is given the option of reviewing a further MPR series in which case the workflow is repeated.

In Step S9, the workflow ends with a report being issued.

### SUMMARY OF THE INVENTION

Certain embodiments of the invention provide a computer system for displaying original and derived images and image series on a user interface, each derived image series comprising a sequence of image frames, the computer system comprising: an application component operable to create a specification for generation of a derived image series from an image data set based on user interface interaction with displayed original or derived images, each derived image series specification providing values for a plurality of visualization parameters including a parameter whose value varies from frame to frame within the derived image series and a plurality of parameters whose values are fixed from frame to frame of the derived image series; and to generate a derived image series by manipulating the medical image data set according to the derived image series specification including quantizing the values of the varied parameter; and a display configured to receive and display the derived image series generated from the derived image series specification.

In certain embodiments of the invention, responsive to user interface interaction, the application selects a parameter 't' that varies from frame to frame and quantizes it, while keeping other parameters constant, so that streaming and caching can be effective. The user interaction part of the application, and the mathematical model behind it, is therefore configured to select and quantize the parameter 't'. For example a user interface instruction "go to a particular 3D point" may need to be decomposed into a change of 't', as distinct from the other parameters, and then the difference between a continuous and a discrete change of the quantized parameter 't' needs to be accommodated. This approach differs from prior art dynamic generation of images in response to user interaction in which there can be no effective caching, since a user's adjustment of visualization parameters will be continuous (not discrete) adjustment of several visualization parameters so that in general cached images will not be useable after each user interface command. This approach also differs from prior art approaches which stream and cache stored images without any interactivity with a user's adjustment of interface controls. We move away from an unconstrained interaction and define one "scroll" variable or other parameter to quantize. The usability benefits that follow from imposing this PACS-like constrained design are significant, but perhaps subtle to appreciate.

The user interaction from which the derived image series specification is obtained can be user interaction with at least one of: an original image, a derived image, an original image series and a previously generated derived image series of the same medical image data set which is used to obtain the derived image series, or a related medical image data set, wherein the related medical image data set may be from the same modality or another modality, or from the same patient, or a different patient.

The application component may be split into first and second parts, for example with a first part in a PACS visualization application and a second part in a 3D visualization application. Alternatively, the application component may be split into two components of the same visualization application. Moreover, the application component may run on a single hardware platform, such as a single server, or may be split into parts running on different hardware, such as separate servers or separate computers. The application component, or each part thereof, may also run on multiple computers, for example two computers connected in a server and client relationship connected by a communication path.

The variable parameter can be defined by an image type classification. The image type classification can be obtained from user interface interaction, e.g. selection from a drop down when initiating a session, such as cross-curve MPR, or in some other way, such as from file attributes of the medical image data set, e.g. DICOM header information.

The value of the variable parameter can be quantized, i.e. discretized, by applying increments from frame to frame from a start value. The incremental variation can be by an incremental value set by user interface interaction. Generally, the variable parameter is set and varied within the series in an arbitrary way having regard to how the variable parameter has been recorded in the medical image data set from which the specification is intended to create the derived image series. For example, the variable parameter may be quasi-continuous in the medical image data set, i.e. recorded in very small increments, compared with the increments defined in the specification for the derived image series to be generated. Generally, the plane of each image frame will also be arbitrary compared with how the data is stored in the medical image data set, for example the plane of each image frame in the derived series may not align with slices in a CT volume data set. For some image types, the variable parameter varies from the start value to an end value, e.g. in the case of parallel MPR. For other image types, the variable parameter varies cyclically seeded from the start value, e.g. in the case of rotation about an axis. In some cases, the variable parameter will have no direct analog recorded in the medical image data set, for example in the case of a curved projection where the curve has not been explicitly recorded in the medical image data set, but is instead created after the fact by an algorithm or human operator.

The application component can beneficially have access to a cache for storing the derived image series according to a caching protocol.

The application component can be distributed between a client and a server, wherein the client is connectable to the server via a communication link and comprises a display operable to display an image series, and wherein the server is operable to transmit the image series to the client for display. The client can be a thin client, variants of which include a "rich client" that has reduced processing capabilities and domain logic, or a "true thin client" that has minimal processing capabilities and domain logic.

The application component can be configured to transmit frames of derived image series from the server to the client as interactive frame versions and final frame versions, wherein interactive frame versions have lower resolution than final frame versions, such that interactive frame versions are transmitted before final frame versions, and final frame versions supplant their interactive frame versions on the client user interface once received.

The application component in some embodiments has access to a server cache and a client cache and manages transmission of a derived image series from the server to the client according to a caching protocol involving the server cache and the client cache. The caching protocol can be configured such that interactive frame versions are not cached in the client cache, whereas final frame versions are cached in the client cache. The caching protocol can be configured such that the server maintains knowledge of the contents of the client cache. The knowledge may be maintained either on an image series level or a frame level or both.

The application component preferably includes a reporting function which is operable to save to the file store relevant work product from user interface interaction with the derived image series. Relevant work product may include user annotations, measurements, parts of or all of the derived image series, and the specification used to generate the derived image series.

The application component can be operable to display image series in time sequence and/or concurrently alongside one another according to a hanging protocol. The way in which the image series are displayed may be at least partly defined by image type. One example of a suitable derived image series is a succession of two-dimensional image frames obtained from multi-planar reformatting (MPR) of the image data set. MPR may have multiple associated image types, for example separate image types for parallel MPR and curved MPR.

Certain embodiments of the invention provide an image acquisition device comprising a computer system as described above.

Certain embodiments of the invention provide a computer-automated image visualization method for displaying image series derived from medical image data sets, the method comprising: providing a file store containing medical image data sets; displaying original or derived images or image series on a user interface, each derived image series comprising a sequence of image frames; creating a specification for generation of a derived image series from an image data set based on user interface interaction with the displayed original or derived images, each derived image series specification providing values for a plurality of visualization parameters including a parameter whose value varies from frame to frame within the derived image series and a plurality of parameters whose values are fixed from frame to frame of the derived image series; generating a derived image series by manipulating the medical image data set according to the derived image series specification including quantizing the values of the varied parameter; and displaying the derived image series on the user interface. The method may further comprise storing results of the user interface interaction with the derived image series including some or all of the derived image series and/or its specification.

Certain embodiments of the invention provide a computer program product bearing the application component as described above.

Certain embodiments of the invention provide an image acquisition device loaded with and operable to execute machine readable instructions for carrying out the method described above.

Certain embodiments provide a computer system for visualising medical images from volumetric data in which:
- the user is free to use one set of controls and user interface elements to manipulate the image in any way they see fit, exactly as they do on a traditional workstation to define a series, where at this stage in the process the images of the series need not be computed, the important point being that the set of parameters for each image in the series is defined at this stage;
- the user can use a different set of controls and/or user interface elements to scroll through images in the virtual series they have just defined to explore the series in a way that there is no possibility for a cache miss if the controls result in requesting an image from the series which has already been computed;
- images from the series are generated according to user demand, rather than a more traditional approach of generating the entire series up front. This allows the first image to be delivered quickly, while retaining good caching performance, since images that are viewed twice do not have to be re-rendered and, in the case of client-server, retransmitted to the client.
- images from the series can also be generated in advance of user demand by "pre-rendering" them according to some predictive algorithm that attempts to decide which images the user may want to see next.

Because this approach defines a series before the series has been rendered and/or been explicitly requested by a user, it can be referred to as a "virtual series". Indeed the "virtual series" may never be computed if the user changes the non-quantized parameter through the user interface before exploring the virtual series. Implementing the virtual series approach is non-trivial, since in many cases it will require imaging parameters to be split into one component that affects the series definition, and another component that affects the image selection.

The concept of a virtual series as described herein is one in which a set of operations defines the virtual series, and another explores it by selecting which image from the virtual series to compute and/or display.

In some embodiments, derived images of the virtual series are generated interactively according to user demand to view said derived images and then cached for later use

In some embodiments, derived images of the virtual series are generated predictively and cached in advance of user demand

In some embodiments: one set of user interface operations allows the user to freely define the specification of a derived image, and said derived image specification is interpreted as defining a virtual series of which the derived image is a member; whilst another set of user interface operations allows the user to select which derived images from a virtual series specification should be computed and/or displayed.

A feature of certain embodiments is where a user interface interaction for generating a specification for a derived image series is indistinguishable from a user interface interaction for generating a specification for a single derived image.

A feature of certain embodiments is where the user interface interaction is configured to present a preferred manipulation of viewable images, said interaction corresponding to the variation of the visualization parameter that varies from frame to frame within the derived image series.

A feature of certain embodiments is where the user interface interaction is configured to discourage or avoid manipulations that alter the plurality of parameters whose values are fixed from frame to frame of the derived image sequence.

A feature of certain embodiments is where the user interface interaction is configured to substitute actions that alter the parameter whose value varies from frame to frame in the derived image sequence, in place of actions that alter the plurality of parameters whose values are fixed from frame to frame of the derived image sequence, for example a "triangulate" command can be implemented to scroll to the nearest MPR plane rather than re-position all MPR planes exactly on the specified point.

A feature of certain embodiments is where the user interface interaction is configured to start or end the generation of derived image sequences in response to changes in the parameters whose values are fixed from frame to frame of the derived image sequence automatically and without requiring user action.

The client component can be operable to allow the user to perform a user interface interaction that results in varying the parameter whose value varies from frame to frame of the derived image sequence, and to display stored frames of the derived image sequence from a cache without re-generating or re-transmitting said images.

A feature of certain embodiments is where the current value of the vector of parameters whose value is fixed from frame to frame of the derived image sequence is used to influence the allocation of caching space to images relating to different vectors of parameters.

A feature of certain embodiments is where the cache is operable to store all or a subset of the frames of the derived image sequence which relate to all or a subset of the distinct value of the quantized parameter whose value varies from frame to frame of the derived image series.

A feature of certain embodiments is where the application component is configured to transmit frames of derived image series from server to client, such frames relating to values of the quantized parameter that varies from frame to frame of the derived image sequence distinct from the currently displayed value.

A feature of certain embodiments is where the values of the quantized parameter are systematically related to the currently displayed value, for example i+n, i-n.

### BRIEF DESCRIPTION OF THE DRAWINGS

Embodiments of the invention are now described by way of example only with reference to the following drawings.
Figure 1 is a schematic drawing of principal elements of a medical imaging product chain.
Figures 2a, 2b and 2c show three examples of derived image sequences.
Figure 3 shows a standard workflow for preparing a sequence of derived images.
Figure 4 is a simplified schematic diagram showing a client-server architecture following a standard PACS system which can host a first embodiment.
Figures 5a, 5b and 5c shows the evolution of the client cache and a counterpart mirror of the client cache located on the server during transfer of a derived image series from server to client.
Figure 6 shows an example workflow according to the first embodiment which is to be compared with the standard workflow of Figure 3.
Figure 7 shows a client-server architecture according to a second embodiment.
Figure 8a shows display of a derived image series on a set of four displays 1, 2, 3 and 4 at a client workstation according to a first use case.
Figure 8b shows display of a derived image series on a set of four displays 1, 2, 3 and 4 at a client workstation according to a variant of the first use case.

### DETAILED DESCRIPTION

First of all, it is noted that the description of the generic prior art system made above with reference to Figures 1 and 2 of the accompanying drawings also forms part of the detailed description of embodiments of the invention.

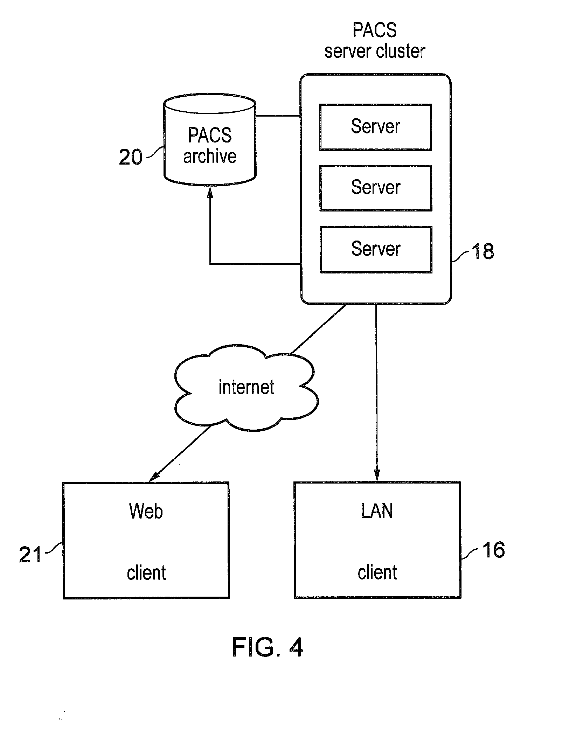

A first embodiment is now described with reference to Figure 4 which is a simplified schematic diagram showing a client-server architecture following a standard PACS system which is suitable for hosting a visualization application implementing the first embodiment. Other embodiments will be implemented on a workstation rather than client-server, so the client-server specific aspects of caching described in this embodiment are non-limitative to the invention. Further embodiments will be implemented on thick-client systems. A PACS archive 20 is connected to a PACS server cluster 18. Web and LAN client computers 21 and 16 are connected to the PACS server cluster 18 via the internet and a hospital network connection respectively. In the following description of the first embodiment we assume a visualization application for generating images is on the server, but the location of the visualization application may be different in other embodiments.

Generally speaking, the common situation is that the server running the visualization application can generate images (i.e. render) much faster than those images can be transmitted to the client. Consequently, the server needs a buffer, e.g. a cache, to store rendered images before they are transmitted to a cache at the client. The logic contained in the visualization application for generating images will therefore include implementation of a caching protocol. The caching protocol is, for example, used to check the cache to establish whether an image is already cached. It is desirable to avoid a server-client-server round trip to check if an image is cached. Therefore, the server keeps a logical mirror of the cache that is held on the client so knowledge of the client cache contents is maintained at the server side. The server hence knows what images are cached at the client and can send either pixel data or an image frame identifier (image ID) for each frame as needed without having to ask the client if it needs it. The transfer of images of a series to the client is now described.

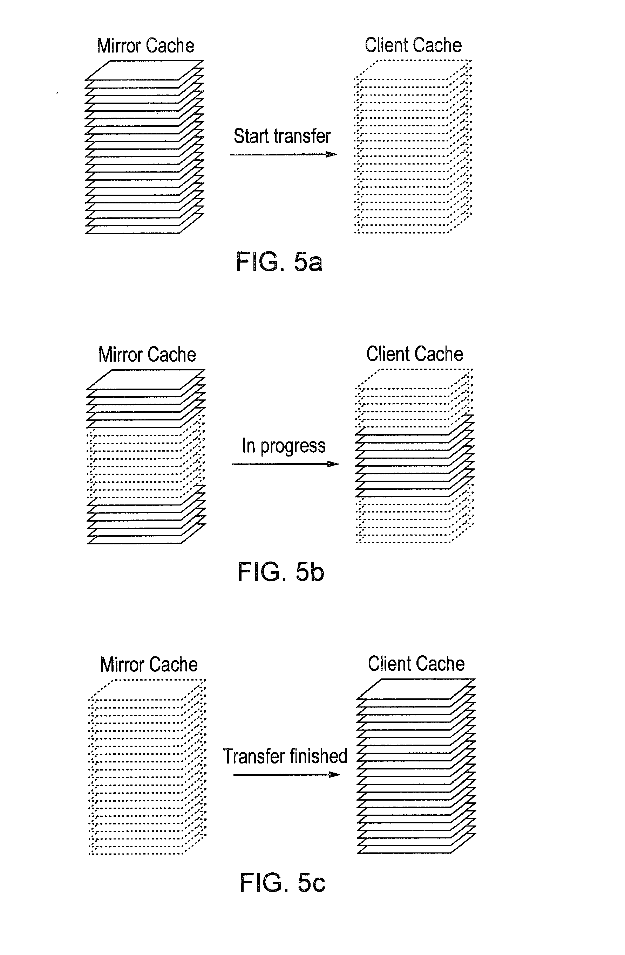

Figure 5a shows the initial situation at the start of data transfer when the server-side mirror cache has all the pixel data and the client cache is empty. The mirror cache thus has to send pixel data to the client to show every frame.

Figure 5b shows the situation some time later, for example a minute later, when an intermediate state has been reached, in which some but not all of the pixel data is in the client cache. In this intermediate state, when requested by the client, the mirror cache at the server either sends only the image ID if already cached at the client, or the pixel data otherwise.

Figure 5c shows the final situation after more time, for example several minutes, when the client cache has all the pixel data. The mirror cache at the server only keeps a record of the image IDs of the frames stored in the client cache and/or a digest of the values of all parameters used to generate the images. When requested by the client, the mirror cache sends only image IDs to the client to enable the client to find the frame in its cache.

A derived image series can be generally defined as a set of parameter variables whose value is common among all series members combined with a single parameter whose value varies from series member to series member, i.e. from image to image in a given series. The approach adopted here is based on identifying one parameter, or degree of freedom, per image type from the many that define any given image type. It is noted that the one parameter identified may not be a parameter conventionally associated with rendering a single image from a volume. For example, in order to define a parallel MPR series, it is necessary to define the series axis, origin and inter-image spacing, and then determine which image to display by projecting the user's specified viewpoint onto this axis to determine the currently visible image from the series. In comparison, without the "virtual series" concept, it is merely necessary to define a viewpoint and viewing direction before rendering the images that make up the series. It will therefore be understood that, although one parameter or degree of freedom is selected to be the series variable, this is only possible because the parameters normally chosen to define the viewing conditions have been refactored into a "virtual series" model.

Image type is a common classification in medical imaging to distinguish between types of derived image series such as planar or parallel MPR images, curved MPR images, 3D volume rendered images and simulated endoscopic images. Image type may be set, for example, by a user at the start of a session, or by an automated algorithm known as a hanging protocol which references the DICOM header, when loading a medical image data set from the PACS archive, or from user interface interaction, such as being inferred from a tool that the user has selected for manipulation of an image being viewed on the display screen, or by a combination of any of these factors.

For parallel MPR, for example, we refer back to Figure 2a, noting that the distinguished degree of freedom is the parameter t which moves the image plane along its normal vector, whereas the other degrees of freedom which are fixed from image plane to image plane are the two positional coordinates and three orientation angles of the sectioning plane. For cross-curve MPR (Figure 2b) and rotational volume rendering (Figure 2c) the parameter t is distance along the vessel axis and angle about the patient axis respectively.

Variation of this distinguished degree of freedom, i.e. the varied parameter, all other degrees of freedom being constant, i.e. fixed parameters, defines a nominal sequence of images, which we may also refer to as frames. Suppose that an image type is defined by five degrees of freedom (A, B, X, D, E) and we choose X as the distinguished one. Then these two images are distinct images of the same sequence:
(A, B, X, D, E), (A, B, X', D, E) when X ≠ X'

And these are four examples of images that are in different sequences than (A, B, X, D, E):
(A', B, X, D, E) when A' ≠ A
(A, B', X, D, E) when B' ≠ B
(A, B, X, D', E) when D' ≠ D
(A, B, X, D, E') when E' ≠ E

The distinguished degree of freedom is modeled as a discrete quantity, even if it is naturally continuous, if necessary by quantizing its set of possible values. The modeling of the parameter as discrete is built into the visualization application, but the details of its quantization (such as the interval and anchor points) are allowed to vary in response to user control. The other degrees of freedom may be modeled as either continuous or discrete. This defines a set of nominal image sequences, and each image sequence contains an ordered collection of images which can be identified by mapping the integers to different quantized values of the distinguished parameter, as follows:
(A, B, Xi, D, E) where i is an integer

In theory the sequence of images defined by Xi could be infinite, but medical images are generally finite, e.g. based on modality constraints. There are two practical kinds of image sequences, linear and cyclic. Linear sequences have a start and end, for example a transverse sectioning plane sweeps from the head to the foot of the patient at 1 mm intervals. Cyclic sequences are finite but presented as infinitely repeating, for example a rotational MIP projection can be presented as rotating 360° around the patient at 1° intervals and repeating indefinitely.

An image sequence is defined by:
- The vector V of parameters other than the distinguished one. In this example V = (A, B, D, E)
- A function Xi = f(i) where i in range [p, q] that maps integers to the distinguished parameter X

This selective imposition of constraints allows caching to operate effectively in a system that generates, transmits, and displays derived images in a client-server configuration, e.g. a thin client configuration.

The implementation of caching is straightforward to a skilled person, but a specific implementation is now described for completeness. The cache needs to distinguish between the many distinct image sequences that may be instanced in the product either concurrently (because they are displayed in different windows) or at different times (because the user varied one of the parameters that define a sequence). Thus in principle the cache needs to perform an associative match of these inputs:
- The vector V of parameters that partially define the sequence
- The function Xi = f(i) that partially defines the sequence

In practice, the vector V is typically embodied in data such as numbers, matrices, tables, etc. Different choices for function f are typically embodied in program code. In a preferred example implementation, different choices of f are represented as data using an enumerated token T. The vector V and the token T are processed with a hash function to generate a key K, which is used to perform an associative match using a hash table or similar data structure. This implementation allows the cache to remain useful if the user changes V to V' and then V' back to V. Other implementations are possible. The cache is also directly indexed by the integer i that defines the image number along the sequence. Since it is beneficial to store the entirety of each image sequence, a possible implementation is to map each cache key K to an expandable array of images indexed by i. However, since the aggregate number of images in all the image sequences that might be cached can be very large, it is also beneficial to include i in the inputs to the hash function that generates key K and map K directly to images using a hash table or similar structure, so that images can then be considered individually as candidates for eviction from the cache.

There are two areas in the imaging chain when the performance of a product that generates derived images may be limited by technical factors and could benefit from the kind of caching proposed here. First, the rate at which derived images can be transmitted from the server to the client in a client-server deployment is typically limited by the available bandwidth between server and client, and the acceptable degree of compression that may be applied to the images while retaining their medical accuracy. Second, the rate at which derived images can be generated may be limited by the computing power available, e.g. the computer processing unit (CPU), graphics processing unit (GPU), memory, and similar computing resources. In addition, these resources may be contended by other potential uses, such as serving other concurrent users of a client-server system.

In the client, caching is applied to every image that is received from the server, or to a subset of those images. This application of caching is highly beneficial. Two possible variations on its implementation are now mentioned in which the logic that determines the need for a new image to be sent is hosted either at the server or the client. Optionally, caching can also be applied in the server to the results of rendering operations. We refer to this in the following as a rendering cache. Before driving the rendering subsystem to generate an image, the server presents the image generation parameters to the rendering cache. If the image is found in the rendering cache, it is available immediately and computing resources are not occupied by rendering it unnecessarily. A rendering cache may or may not provide benefits depending on the performance characteristics of the rendering functions of the server.

If the logic that determines the need for a new image runs on the client, then the client presents the image generation parameters to a cache held on the client before asking the server for the image. If the client cache registers a hit, the image is available immediately and the server is not asked to re-generate it. This avoids the latency of communicating with the server, the use of processing resources on the server, and the time that would be needed to transmit the image from server to client.

If the logic that determines the need for a new image runs on the server, then the server holds a logical mirror of the cache that is held on the client. This means that the server and the client hold two identical image caches, except that only one of the mirror cache on the server and the client cache will need to store the pixel data of any particular image. The server presents the image generation parameters to its logical cache before causing an image to be rendered and transmitted. If the system is configured such that pixel data is stored at the client, when the logical cache registers a hit, the server sends to the client a small data packet containing the cache key K. The client can then use the cache key K to access the pixel data of the image. This approach avoids the latency of communicating between server and client, unnecessary use of processing resources on the server, and the time needed to transmit the image from server to client (since the description of the image that is transmitted instead is small).

The rendering cache on the server, if present, is filled when an image is rendered. Images may therefore appear first in the server's rendering cache and then later on the client's cache, if rendering has higher throughput than transmission.

The cache on the client is preferably supplemented by a logical mirror of the client cache at the server which is distinct from the server cache. Once the images are on the client's cache, and/or the server's mirror of the client cache if present, there is no benefit in having them also in the server's rendering cache and they can be deleted from there. In a preferred embodiment, the server's rendering cache is small and serves as a buffer between rendering and transmission, while the image cache on the client is preferably as large as permitted by the freely available memory on the client.

When the image generation logic is on the server, the rendering cache and the server's mirror of the client cache are one and the same. Alternatively, when the image generation logic is on the client, and hence no server side mirror of the client's cache is needed, provision of a rendering cache on the server can be beneficial.

Two specific conditions that cause images to be generated and cached are as follows. First, an image is generated and cached when the user manipulates the imaging parameters V and i to request a specific image. For example if the user manipulates the application to display a particular MPR sectional image, that image is cached. Second, images are generated and cached automatically for values of i-j and i+j, where j in [1, ∞]. For example when the user manipulates the application to display a specific MPR image, after that image is cached, additional images are cached which neighbor that image on either side (or only on one side if the scrolling direction can be predicted), and the series is progressively built up over an expanding neighborhood seeded from the initially displayed frame in the series until the entire derived series is cached.

The first type of caching is a type of reactive caching. The second type of caching is an improved type of predictive caching. Rather than attempt to predict from first principles a set of neighboring images of the currently displayed image, the visualization application uses the concept of image type or series type. The visualization application knows the image type currently being manipulated and for each image type has a definition which specifies the single degree of freedom and associated variable parameter i which is to be discretized. The concept of a neighboring image for the purpose of caching is then a straightforward matter of incrementing or decrementing the quantized index i by an amount which could be a default amount, an amount inferred from recent user activity on the user interface, or an amount explicitly set by the user. This allows the whole of an image sequence to be cached reliably and efficiently.

In embodiments of the invention, the workflow identifies and discretizes parameter X so that caching will be efficient, whatever caching protocol is used, e.g. whether it be reactive and predictive. With a conventional approach to browsing a volume, a user scrolling up and down in an MPR mode would convert mouse movements into floating point changes to the MPR plane position so that, if for example reactive caching were used, the chance of revisiting an image exactly, and thus making a cache hit, would be vanishingly small. A conventional approach of avoiding this problem when browsing is to provide a "movie mode" where the user has to take explicit steps to set up a series, such as a coronal MPR or rotating volume view, and then hits a "play" button to initiate its computation as a series. Since the computer is generating the increments from image to image in the series and knows that it is a movie, it can cache effectively. The "virtual series" approach differs from either of these conventional approaches invention in that it provides a browsing-based user interaction with free exploration of the data which can also cache effectively.

As mentioned earlier, image sequences can be linear or cyclic. Caching works somewhat differently in each of these cases. In linear series, images at i-j and i+j are cached until the limits of the sequence are reached in each direction. In cyclic series, images at (i-j) mod n and (i+j) mod n are cached, where n is the total number of images in the cyclic sequence, so that the image neighborhood wraps around the series correctly.

It is beneficial to transmit a whole series, i.e. frames covering the full range of parameter t, to the client so that, once cached at the client, the user can navigate through the entire extent of the series with a high update rate in frames per second. In many situations this will be achievable, especially if compression is used to reduce the size of cached images on the client. However, it will not always be possible to cache the whole extent of all the series that are visited during a session, and some method of evicting images from the cache is needed, to make room for other images. Some possible schemes for evicting individual images or whole image series from the cache are as follows:
- *Least Recently Cached:* The image or series with the oldest creation time stamp is evicted.
- *Least Recently Viewed*: The image or series with the oldest time stamp of being displayed is evicted.
- *Favoring active series:* The images belonging to series that have been viewed and subject to user interface manipulation (e.g. have received mouse focus, direct user input, or indirect changes due to user input) are retained while other images are evicted.
- *Favoring current series:* The series belonging to images whose parameters V are currently in effect are retained, while the images relating to previous values V', V" etc. of the parameters are evicted.

The caching scheme can also benefit from use of an image classification between interactive images and final images, only caching the latter. Interactive images are lower quality images that can be generated and displayed immediately in response to user input, so that the user gets immediate feedback for their actions, and in particular their user interface manipulations. Specifically, interactive images may be rendered using an approximate, but faster method, and/or for transmission they may be compressed with a high degree of data loss (lossy compression), so that they can be transmitted quickly to the client through limited bandwidth. Final images are perfect quality images, or at least high enough quality for reliable diagnosis, so need to meet certain minimum standards of rendering quality and transmission fidelity. For example the application may require perfect reproduction (lossless compression) or a low degree of data loss in lossy compression. Final images are generated and displayed to provide the user with the diagnostic information they need, and back fill or supplant the lower quality interactive images once available at the client.

There are a number of choices regarding the data type of the image that is cached on the client.

Medical images are commonly grayscale with an original bit depth of 12 bits. Common display devices can typically reproduce only 8 bits of intensity gradation. Common practice is to apply a "bias and slope" transform, or an arbitrary transform, to the 12-bit image in order to arrive at an 8-bit image that can be displayed. In the present invention this transform could be applied on the server resulting in 8-bit images cached on the client, or the 12-bit images could be transmitted and cached on the client such that the transform is applied on the client. Both approaches have merit for different use cases.

Derived images in medical applications are commonly clipped by the viewport where they are displayed, i.e. clipped to the specification of the display. For example, a curved section of a vessel may extend to 400 x 1500 pixels in the image data set but, when displayed in a 512 x 512 pixel viewport, only a 400 x 512 pixel portion of that is visible. One embodiment would cache the entire 400 x 1500 image on the client so that subsequent panning around the image can be achieved quickly. Another embodiment would cache only the contents of the 512 x 512 viewport, so that the cache is filled more quickly to show the initial image, and accept some latency if panning then follows.

Medical images are commonly overlaid with lines and/or text drawn by the imaging application to convey information about the patient, the imaging method, geometric landmarks, key features of the image, etc. In one embodiment, medical images are cached at the client without these decorations. The decorations are transmitted by separate means, preferably as a description of the text and geometric objects, and then drawn over the image at the client. This allows the overlays to be updated without re-transmitting the medical image, which is a common operation, and avoids distorting the lines or the text if lossy image compression is applied.

Thin client applications in medical imaging generally use a form of compression to reduce the amount of data that needs to be transmitted per image, and thus allow images to be transmitted more quickly to the client. It is beneficial in the present case to cache the compressed representation of images on the client, since this uses the capacity of the cache more efficiently and since the time to decompress images is generally short. The degree of compression that is acceptable in different medical application varies widely. Some relevant factors are the clinical application area, the professional bodies that are influential in the region where the product is used, legal requirements that may apply, policies set by healthcare providers, and the judgment of healthcare professionals. One common requirement is to support perfect reproduction of images (lossless compression). Another common requirement is to support images compressed and reproduced according to an established standard, such as JPEG, with a controlled amount of data loss (lossy compression). It may be advantageous for the system to be configured to allow the user to set the desired compression level of final images, and to always deliver final images using that quality level. On the other hand, for interactive images for which lossy compression may be acceptable, the degree of compression can be automatically selected and varied to meet performance metrics, such as maximum permitted latency times. Alternatively, the degree of compression for interactive images may be set by the user also. If a non-perfect quality level has been set for the final images, it is preferred not to refine them to even higher quality, but rather use the available time and network bandwidth to cache additional images in the series at the specified non-perfect quality level for final images.

The visualization application presents to the user original images and derived images. The operation with regard to original images is conventional, and will not be discussed further. With respect to derived images, the visualization application presents the user with a choice of image series. Rather than choosing an MPR view, the user chooses a parallel MPR series view or a radial MPR series view according to the clinical task at hand. The choice can be further streamlined by packaging collections of image series that work effectively towards a clinical task in assemblies called protocols. For example a protocol for diagnosis of vascular ailments may contain parallel MPR, curved MPR, cross-curve, tangent to curve, and rotational 3D image sequences.

Within an image sequence the tools and operations available to the user are subdivided into two groups: (i) Operations that vary the index i of one or more series; and (ii) Operations that vary the definition V of one or more series. The first group of operations results in selecting different images from the same series for display, and therefore results in fast updating once the relevant images are cached. The second group of operations changes the definition of series so that any images already cached according to the old definition are no longer relevant, and new images need to be cached according to the new definition. Therefore these operations do not benefit from improved performance through caching, and cause additional computation.

For every image series, one tool is identified that changes the quantized index i directly. This is called the Scroll tool and typically for medical imaging applications it is bound to the action of dragging the mouse with either the left or the right button down. Additionally the Scroll tool may be bound to the scroll operation of a wheel mouse or touch pad that supports scrolling. The operation of the scroll tool depends on the series type. Some examples are as follows. If the series is parallel MPR, the scroll tool moves the plane in parallel. If the series is radial MPR, the scroll tool rotates the plane about an axis. In a cross-curve series, the scroll tool moves the plane along the curve. With a rotational 3D series, the scroll tool virtually rotates the patient around their vertical axis.

The operation of scrolling thus presents a very natural form of navigation to the user according to the type of series they are using. Instead of having to learn which of the many tools manipulates the imaging parameter in the desired way, the user achieves most of their tasks using the default scroll operation of each type of sequence. Scrolling of course benefits from caching so that the user perceives a high update rate, once images are cached, irrespective of network bandwidth and irrespective of the quality and compression settings that the user has set for final images.

A further benefit is that scrolling is fully repeatable. In other words if the user scrolls back and forth, exactly the same images are seen. It is possible for the computer to check that all the images of the series have been seen (or at least displayed) and to emit warnings when images in the series remain unseen. This is a further benefit of the novel concept of a series being made up of a finite set of derived images even though the variable parameter i may be so finely stepped compared with the resolution of the volume data set to be effectively continuous, i.e. quasi-continuous. Because a defined and finite sequence of images is being presented, it is straightforward to take a permanent copy of the series in a standard medical imaging data format such as DICOM.

In addition to the Scroll tool, some other tools may be implemented such that they vary the index i of one or more series. One such candidate is the Triangulate tool. This tool causes several image series to display a point identified by a mouse click on one of the series. A possible implementation of Triangulate is to vary the parameters V of all the affected series so they include images that contain the point exactly. However, this negates the value of all currently cached images. A preferred implementation is to vary the index i of all the affected series so that they display the point approximately, by revealing the existing image that is closest to the point in each series. This implementation preserves the value of cached images.

Some operations that typically vary the vector V of imaging parameters are the following:
- Changing the inclination of the sectioning plane of a parallel MPR series
- Changing the position and inclination of the axis of a radial MPR series
- Changing the curve definition related to a cross-curve MPR series
- Changing the color, opacity, or brightness properties of a rotational 3D or MIP series
- Changing the inclination of the camera of a rotational 3D or MIP series
- Changing the field of view, flight path, or looking direction of a virtual endoscopic camera

These are just some examples of operations that change the vector V. When the user performs one of these operations, cached images relating to a previous value of V" of that vector are no longer useful for accelerating the display of images on the client. These images may be evicted from the cache immediately, or eventually in response to the eviction policy in use. There is a chance that these images might once again become useful if the user later changes the value of V back to V". This would be expected to happen often if the user interface includes an undo function for these operations. When the user performs one of these operations the system starts generating and caching new series of images according to the new value of V. While this is happening the system may only be able to display interactive images to the user. However, once a substantial number of images have been cached, the system once again allows fast update of final quality images, as already explained.

In summary of this embodiment, a user is free to adjust the totality of imaging parameters that make up vector V as in a conventional 3D visualization application and the characteristics of the system when the user is adjusting vector V are equivalent to the characteristics of conventional systems. However, when varying the index i in the context of a single derived series while vector V is not changed, the system can perform as well as a conventional system that already has the required series stored, although this is only generally true for client-server embodiments once the full resolution series has reached the client. Before this, some intermediate quality images may have been provided.

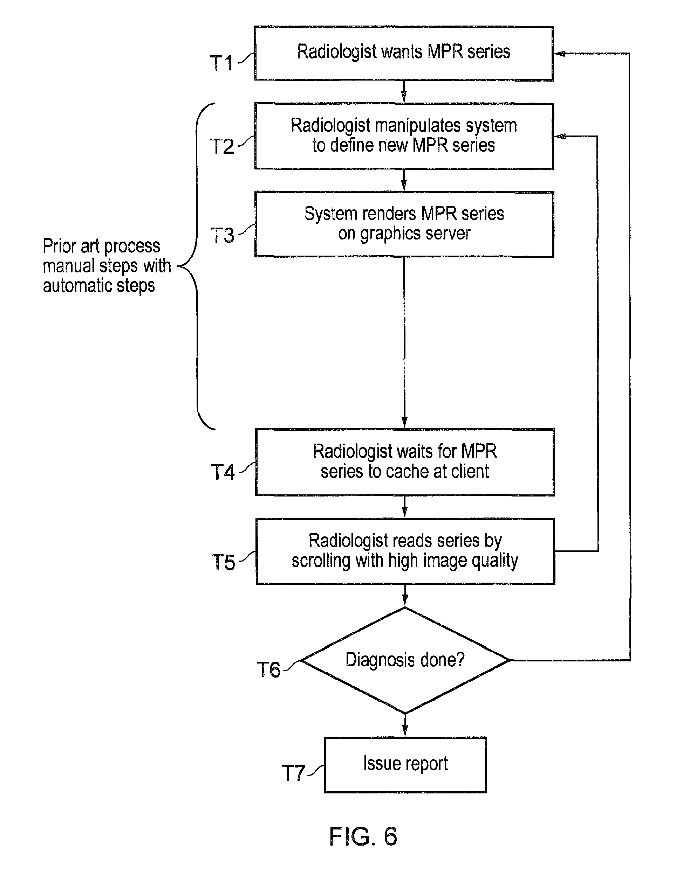

Figure 6 shows an example workflow according to the first embodiment which is to be compared with the standard workflow of Figure 3. The example workflow, like the standard workflow, is for preparing a sequence of derived images for MPR, such as would be applicable to the MPR examples of Figures 2a and 2b. This example workflow is a specific example of the general derived image workflow shown in Figure 1.

In Step T1, the sequence initiates with a radiologist wanting to have an MPR series prepared for his or her analysis (same as Step S1).

In Step T2, the radiologist uses the client application to manipulate the viewed image in a way which either explicitly results in the radiologist defining a desired MPR series, or allows the client application to infer an MPR series, i.e. a derived image series of a particular image type, which the radiologist would like to view in order to conduct a diagnostic analysis. The radiologist's interaction with the user interface explicitly or implicitly defines a specification for the visualization, namely the viewing parameters which are aggregated into token T and vector V, where the meaning of vector V and token T are as described above.

In Step T3, the specification of the desired MPR series is received by the graphics server via the PACS server and the MPR series is generated and sent to the client via the PACS server.

In Step T4, the radiologist waits for the MPR series to cache at the client, i.e. waits for the MPR series data to transfer over the LAN or internet (same as Step S6).

In Step T5, the radiologist reads or views the series by scrolling interactively up and down by varying the parameter t (i.e. index i). Further user interaction with the user interface may cause the process flow to jump back to Step T2 if the user interaction is interpreted to be a request to redefine the token T and/or vector V. Once the radiologist has concluded the study of the MPR series loaded in Step T1, the process flow moves to Step T6.

In Step T6, the radiologist is given the option of reviewing a further MPR series in which case the workflow is repeated (same as Step S8).

In Step T7, the workflow ends with a report being issued (same as Step S9).

An example case of where the vector V is changed at Step T5/T2 is when a user through the user interface adjusts the inclination of the sectioning plane, e.g. in planar MPR, to indicate that he or she wishes to see a slightly different series at the new plane angle. In this case, the visualization application transparently repeats steps T2 to T4 such that the adjusted series automatically appears on the display.

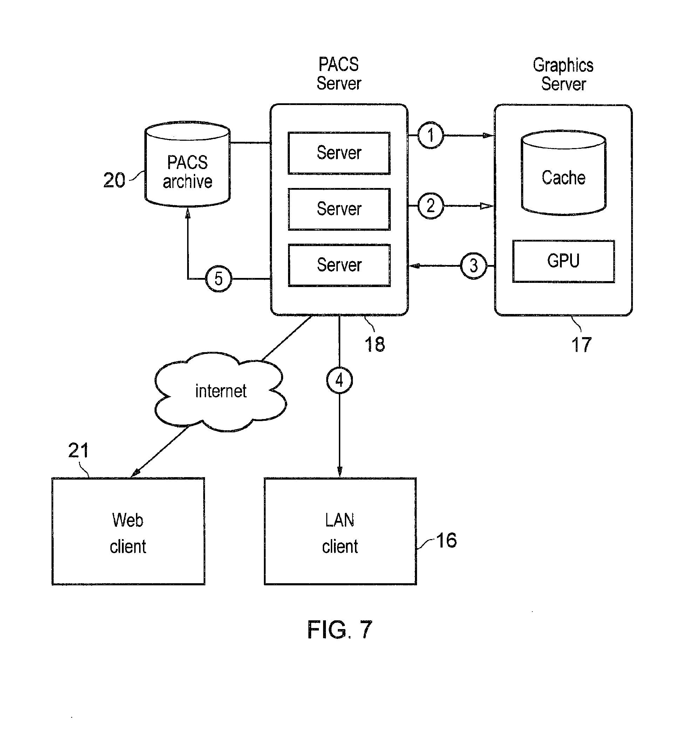

Figure 7 shows a client-server architecture according to the first embodiment, which is a modification of a standard PACS system client-server architecture as shown in Figure 2.

As in the standard client-server architecture, the client is a workstation 16, 21 connected to the main PACS servers and file store 18, 20 via a network connection, which may be a high speed network connection in the case of a LAN client 16 and an internet connection through general telecoms infrastructure in the case of a web client 21. Differing from the standard approach, an additional graphics server 17 is provided, which is connected to the PACS server 18 by a high speed connection. For example, the servers 18 and 17 may be hosted in adjacent server racks and connected by optical fiber data links. The graphics server is optimized for graphics processing, and in particular processing of volume data set by rendering, such as volume rendering or multi-planar reformatting. The graphics server is schematically shown as containing a GPU and a memory cache.

The purpose of the graphics server is to create derived image series rapidly under instruction from the PACS server. In this embodiment, the application logic for selecting a series type (token T), parameters of the series (vector V) and selecting an image to be displayed (index i) are preferably implemented in the PACS server or the PACS client application, but other embodiments may deviate from that approach.

The flow of data between the PACS server and the graphics server occurs in five steps numbered in sequence 1 to 5 in Figure 7.

In Step 1, the PACS server pushes modality images to the graphics server where they are cached until the read.

In Step 2, the PACS server sends to the graphics server a description of the series to be generated.

In Step 3, the graphics server sends to the PACS server the generated series.

In Step 4, the PACS server sends the series to the client workstation for display, in the same way as it would send a series made up of original images.

In Step 5, the PACS server stores some of the derived image series generated during the session in the PACS archive.

Each of these steps is now described in more detail.

In Step 1, the PACS server pushes the DICOM series generated by the modality into the graphics server, where they are cached until the study is read. This step has to take place at any time between acquisition and reading. One example policy is to push studies to the graphics server as soon as they are acquired. Another policy is to push them as soon as they appear in the work list of the PACS server, which may range from a few minutes to a few days before reading. The graphics server has a limited cache space for storing modality images, which corresponds to a time window of a few days to a few weeks, depending on the server specification and the volume of data generated daily by the facility. For example 500GB cache in the server and 50GB/day scan volume means a 10-day window cached in the graphics server. This window is not intended to be long enough to hold prior studies until the time of a follow-up visit. The PACS server is expected to locate and push priors again to the graphics server at the time they are needed in a follow-up exam (assuming that further use of derived images on a previously acquired and reported study is desirable).

In Step 2, the PACS server sends to the graphics server an instruction to generate an MPR series. If the generation of the series is initiated by a PACS hanging protocol, this step can take place at any time before reading. Two example policies are when the study appears in the PACS server work list (at which point the protocol could be automatically chosen), or at the moment the study is presented to the radiologist and they select or confirm the protocol. If the series is requested by the radiologist interactively during a read, this step takes place at the moment the radiologist defines the series. The instruction that the PACS server gives to the graphics server includes the exact set of DICOM images that are to be interpreted as the volume from which the MPR series will be resampled as well as the co-ordinates, orientation, size, thickness, and other parameters of each image to be generated in the derived MPR series.

With respect to co-ordinates and other parameters of images, we propose that logic in the client workstation computes the parameters of each image to be generated, sends them to the PACS server which instructs the graphics server simply to obey the parameters and generate the derived image series accordingly. Alternatively, the logic for selecting an/or computing image parameters may reside in the PACS server.

It also allows the application running on the client to be re-written to provide a variety of desired user interfaces, for example providing a circular overlay to create a radial series, without needing to make any changes at the PACS server. For example, the PACS can present a user interface allowing the user to manipulate the series type, set the parameters that define a series, such as the inclination of an MPR plane, and to select an image from the virtual series to display in a way similar to the first embodiment described above.

The protocol of the graphics server can be DICOM-based. Since there is no standard DICOM message to describe a request to generate a derived image series, the request is encoded in a non-standard combination of standard DICOM tags. Specifically, the PACS server sends to the graphics server one DICOM message for each image to be generated. This has a complete set of tag information but no pixel data. The tag information contains the parameters of the image to be generated (coordinates, direction cosines) and references to the source images (frame of reference, image unique identifier (UIDs) or key image note). The graphics server returns a DICOM message for each image that includes the tags exactly as received plus the generated pixel data for the image. This interface allows the PACS precise control over the DICOM tags of the generated images. In alternative embodiments DICOM compatibility may not be required, for example a proprietary protocol may be used.

In Step 3, the graphics server sends the generated series to the PACS server. This starts as soon as the first image is generated, moments after Step 2. For efficiency, the PACS server preferably asks for all the images in a series at once. (Alternatively, the PACS server could request the image series in blocks on demand as the user browses.) Upon receiving the request, the graphics server will generate the entire series, typically in a few seconds, and will send the entire series to the PACS server at the maximum speed permitted by the link. Thus, for efficiency, Steps 2 and 3 should pass entire series (even if the client workstation is only pulling a few images.) The simplest way for the graphics server to send the series to the PACS server is through DICOM push. Should this be a bottleneck, for example because of queuing issues with other incoming DICOM data, an alternative is for the graphics server to deposit the images as DICOM files on a nominated common Internet file system (CIFS) file share, or to transmit them through a proprietary protocol. In further alternative embodiments a proprietary data link may be used for data transfer which does not involve transfer of DICOM files.

In Step 4, the PACS server sends the images to the client workstation where they are displayed. The images generated by the graphics server are treated just like the modality images for the purpose of transmission to, and display on, the client. Standard compression, background transmission, and client-side caching techniques can be used in the transfer. Specifically, the PACS server is responsible for compressing the images before transmission, and to implement any compression policy, for example adhering to a minimum quality level for lossy compression, or sending perfect images in certain circumstance such as measurement. (Alternatively, the graphics server could transmit the series to the PACS server as compressed images.)

In Step 5, the PACS server stores a subset of the series generated by the graphics server and reviewed by the operator at the client workstation into the PACS archive, as part of the permanent medical record. This step takes place when a study is reported. At that point, the PACS server is temporarily holding all the additional series generated by the graphics server as part of the read. There is no need to ask the graphics server to do any additional operation, or to bring images from the client workstation back to the server, in order to achieve this.

An example of using the second embodiment is now described with reference to what is shown on the set of displays at a client workstation according to a suitable hanging protocol.

A patient presented with abdominal pain and a history of pancreatic disease. They received a multidetector CT scan (MDCT) with two acquisitions before and after giving the patient a suitable contrast agent, namely a pre-contrast scan of the whole abdomen at 1 mm thickness and 800mm extent followed by a post-contrast scan of the whole abdomen at 1 mm thickness, and 800mm extent. The PACS visualization application has a "General abdomen" hanging protocol which matches the characteristics of this case.

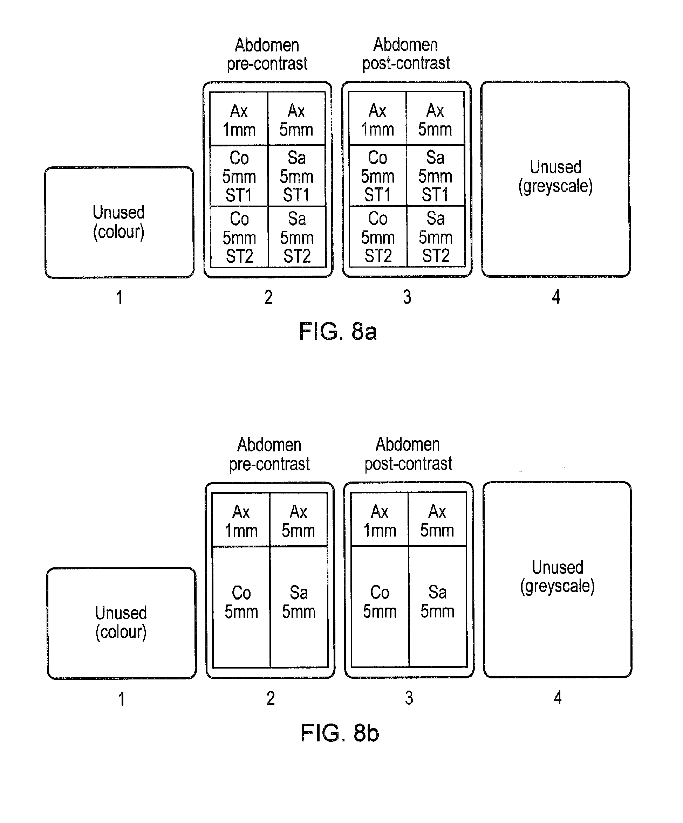

Figure 8a shows schematically a set of four displays 1, 2, 3 and 4 at the client workstation. The protocol presents the case to the radiologist as follows. (We show an unrealistic 2x3 tiled layout in displays 2 and 3 to keep the diagrams simple.) By virtue of the graphics server, the PACS hanging protocol is able to display immediately all these desirable series, without requiring any action from a modality technician or from the radiologist to set them up. The "Ax 1mm" series are generated by the modality, and the remaining ten are generated by the graphics server. The protocol presents 5mm average-intensity slabs in the axial, coronal, and sagittal planes in order to deliver a quick overview of the case. In order to fit the 800-pixel vertical resolution of the coronal and sagittal reformats into standard 512-pixel square series, the protocol automatically splits the scan into two stations. "ST1" is the upper section of the abdomen and "ST2" is the lower.

Figure 8b shows a variant of the same use case suitable when the PACS server and related infrastructure can handle rectangular CT images. In this case, the coronal and sagittal series can be displayed unbroken as illustrated schematically.

All that the radiologist needs to do to read the case is to concentrate on the most appropriate series and scroll (browse). The series are 16-bit DICOM CT, so all the tools that the PACS visualization application provides for CT series are usable: magnifying glass, WW/WL, linear and HU measurement, annotations, etc. Additionally, the radiologist is able to perform a "triangulate" gesture such as left-click on a point of interest and all the series (in both scans, assuming the modality has used the same DICOM frame of reference) are browsed to show the same 3D point. The PACS application can easily be written to create overlays that show the mutual location of points or images in 3D, and allow the user to change interactively the parameters of generated series, such as camera parameters of 3D rotation, plane angle and position for MPR, etc. In this way 3D navigation in the PACS workstation can be as powerful as it is in a 3D workstation.

The variation of vector V and token T according to the first or second embodiments can be activated by the visualization application in several ways. A first example is where it can be activated automatically as part of a hanging protocol. This allows a hanging protocol to present series that the modality did not generate, such as coronal and sagittal series. A second example is where it can be activated interactively upon the user's request. This allows the user to ask for series with different extent, slice spacing, orientation, or field of view. This option may be particularly useful for home working to cope with severely limited bandwidth client-server communication.

The visualization application can apply any desired policy for the preservation of derived series. A simple policy would be to preserve all series generated in the course of a read, including both those generated automatically by the hanging protocol and those generated interactively. Of course this policy would interact well with hanging protocols that generate fewer series by default. A more storage-efficient policy would be to preserve only derived series marked by the user, either explicitly or implicitly by the presence of measurements or annotations. One particularly efficient storage approach enabled by the "virtual series" concept is to store only the set of parameters that defined the series, together with the original images, which is sufficient to allow the series to be recreated whenever desired. In the remote access scenario, derived series are held in the PACS server during the read, and therefore preserving them does not raise any additional challenges. It is not necessary to send any bulky image data back upstream from the user's home PC to the PACS server. The visualization application can choose the policy of preserving either the perfect version of the series, or the lossy compressed version that was transmitted to the radiologist, or both.

Another policy would be to preserve only the parameters that define a generated series, namely the token T and vector V, instead of the pixel data. This allows series to be preserved instantly and with low storage requirements, since said parameters are small. To reproduce the series, the parameters have to be fed again to the graphics server to generate the images.

Embodiments of the present invention will be described hereinafter and in the context of a computer-implemented system, method and computer program product which may be stored on a non-transitory medium. Although some of the present embodiments are described in terms of a computer program product that causes a computer, for example a personal computer or other form of workstation, to provide the functionality required of some embodiments of the invention, it will be appreciated from the following description that this relates to only one example of some embodiments of the present invention. For example, in some embodiments of the invention, a network of computers, rather than a stand-alone computer, may implement the embodiments of the invention. Alternatively, or in addition, at least some of the functionality of the invention may be implemented by means of special purpose hardware, for example in the form of special purpose integrated circuits (e.g., Application Specific Integrated Circuits (ASICs)).

Embodiments have been described implementing the virtual series approach on client-server platform. However, other embodiments are implemented on thick client and workstation embodiments. In all these platforms, the virtual series approach can be beneficial for avoiding unnecessary re-rendering of frequently viewed images and presenting a familiar "series-oriented" model of application behaviour to the user.

While certain embodiments have been described, these embodiments have been presented by way of example only, and are not intended to limit the scope of the inventions. Indeed, the novel methods, computers and computer program products and devices described herein may be embodied in a variety of other forms; furthermore, various omissions, substitutions and changes in the form of the methods and systems described herein may be made without departing from the spirit of the inventions. The accompanying claims and their equivalents are intended to cover such forms or modifications as would fall within the scope and spirit of the inventions.

## Claims

1. A computer system for displaying original and derived images from one or more virtual series on a user interface, each virtual series defining a sequence of image frames, the computer system comprising:
a first application component operable to create a specification for each virtual series from an image data set, each virtual series specification providing values for a plurality of visualization parameters and including a parameter whose value varies from frame to frame within the derived image series and a plurality of parameters whose values are fixed from frame to frame of the derived image series;
a second application component operable to select the image to be displayed from the virtual series based on user interface interaction;
a third application component operable to generate derived images according to the virtual series specification and including quantizing the values of the varied parameter; and
a display configured to receive and display the derived images generated from the virtual series specification.

2. The system of claim 1, wherein the application component has access to a cache for storing the derived image series according to a caching protocol.

3. The system of claim 1 or 2, wherein the application components are distributed between a client and a server, wherein the client is connectable to the server via a communication link and comprises the display, and wherein the server is operable to transmit the image series to the client for display.

4. The system of claim 3, wherein the application components are configured to transmit frames of derived image series from the server to the client as interactive frame versions and final frame versions, wherein interactive frame versions have lower resolution than final frame versions, such that interactive frame versions are transmitted before final frame versions, and final frame versions supplant their interactive frame versions on the client user interface once received.

5. The system of claim 3, wherein the application components have access to a server cache and a client cache and manage transmission of a derived image series from the server to the client according to a caching protocol involving the server cache and the client cache.

6. The system of claim 5, wherein the caching protocol is configured such that the server maintains knowledge of the contents of the client cache.

7. The system of any preceding claim, wherein the variable parameter is defined by an image type classification.

8. The system of claim 7, wherein the image type classification is obtained from user interface interaction.

9. The system of claim 7, wherein the image type classification is obtained from file attributes of the medical image data set.

10. The system of any preceding claim, wherein the value of the variable parameter is quantized by applying increments from frame to frame from a start value.

11. The system of any preceding claim, wherein the application components have a function operable to save to the file store relevant work product from user interface interaction with the derived image series.

12. The system of any preceding claim, where a user interface interaction for generating a specification for a derived image series is indistinguishable from a user interface interaction for generating a specification for a single derived image,
and/or
where the user interface interaction is configured to present a preferred manipulation of viewable images, said interaction corresponding to the variation of the visualization parameter that varies from frame to frame within the derived image series,
and/or
where the user interface interaction is configured to discourage or avoid manipulations that alter the plurality of parameters whose values are fixed from frame to frame of the derived image sequence,
and/or
where the user interface interaction is configured to substitute actions that alter the parameter whose value varies from frame to frame in the derived image sequence, in place of actions that alter the plurality of parameters whose values are fixed from frame to frame of the derived image sequence, for example a "triangulate" command can be implemented to scroll to the nearest MPR plane rather than re-position all MPR planes exactly on the specified point,
and/or
where the user interface interaction is configured to start or end the generation of derived image sequences in response to changes in the parameters whose values are fixed from frame to frame of the derived image sequence automatically and without requiring user action,
and/or
where the client component of the application is operable to allow the user to perform a user interface interaction that results in varying the parameter whose value varies from frame to frame of the derived image sequence, and to display stored frames of the derived image sequence from a cache without re-generating or re-transmitting said images,
and/or
where the current value of the vector of parameters whose value is fixed from frame to frame of the derived image sequence is used to influence the allocation of caching space to images relating to different vectors of parameters,
and/or
where the cache is operable to store all or a subset of the frames of the derived image sequence which relate to all or a subset of the distinct value of the quantized parameter whose value varies from frame to frame of the derived image series,
and/or
where the application component is configured to transmit frames of derived image series from server to client, such frames relating to values of the quantized parameter that varies from frame to frame of the derived image sequence distinct from the currently displayed value,
and/or
where the values of the quantized parameter are systematically related to the currently displayed value, for example i+n, i-n.

13. An image acquisition device comprising a computer system according to any preceding claim.

14. A computer-automated image visualization method for displaying image series derived from medical image data sets, the method comprising:
providing a file store containing medical image data sets;
displaying original or derived images or image series on a user interface, each derived image series comprising a sequence of image frames;
creating a specification for generation of a derived image series from an image data set based on user interface interaction with the displayed original or derived images, each derived image series specification providing values for a plurality of visualization parameters including a parameter whose value varies from frame to frame within the derived image series and a plurality of parameters whose values are fixed from frame to frame of the derived image series;
generating a derived image series by manipulating the medical image data set according to the derived image series specification including quantizing the values of the varied parameter; and
displaying the derived image series on the user interface.

15. The method of claim 14, further comprising storing results of the user interface interaction with the derived image series including some or all of the derived image series and/or its specification.

16. A computer program product bearing one or more of the application components of any of claims 1 to 12.

17. An image acquisition device loaded with and operable to execute machine readable instructions for carrying out the method of claim 14 or 15.
